Europäisches Patentamt

European Patent Office

Office européen des brevets

(11) Veröffentlichungsnummer: **0 582 201 A2**

# (12) EUROPÄISCHE PATENTANMELDUNG

(21) Anmeldenummer: **93112095.0**

(51) Int. Cl.5: **C07D 317/36**

(22) Anmeldetag: **29.07.93**

(30) Priorität: **05.08.92 DE 4225870**

(43) Veröffentlichungstag der Anmeldung:
**09.02.94 Patentblatt 94/06**

(84) Benannte Vertragsstaaten:
**BE CH DE FR GB IT LI NL**

(71) Anmelder: **BASF Aktiengesellschaft
Carl-Bosch-Strasse 38
D-67063 Ludwigshafen(DE)**

(72) Erfinder: **Teles, Joaquim Henrique, Dr.
Maxstrasse 65
D-6700 Ludwigshafen(DE)**
Erfinder: **Rieber, Norbert, Dr.
Liebfrauenstrasse 1c
D-6800 Mannheim 51(DE)**
Erfinder: **Harder, Wolfgang, Dr.
Bergwaldstrasse 16
D-6940 Weinheim(DE)**

(54) **Verfahren zur Herstellung von Glycerincarbonat.**

(57) Verfahren zur Herstellung von Glycerincarbonat, indem man Glycerin mit Kohlenmonoxid und Sauerstoff in Gegenwart von Katalysatoren der I., II. und VIII. Nebengruppe des Periodensystems der Elemente bei Temperaturen von 0 bis 180°C umsetzt sowie deren Umsetzung zum Glycidol durch Reaktion bei Temperaturen von 100 bis 300°C und Drücken von 0,001 bis 5 bar an Alkali- und/oder Erdalkalimetallsalzen.

EP 0 582 201 A2

Der vorliegende Erfindung betrifft ein Verfahren zur Herstellung von Glycerincarbonat durch Umsetzung von Glycerin mit Kohlenmonoxid und Sauerstoff in Gegenwart von Katalysatoren der I., II. und VIII. Nebengruppe des Periodensystems der Elemente sowie deren Umsetzung zu Glycidol bei erhöhten Temperaturen an Alkali- und/oder Erdalkalimetallsalzen.

Aus der DE-A-22 22 488 und der DE-A-22 65 228 sind Verfahren zur Herstellung von Glykolcarbonaten aus Glykolen mit $CO/O_2$ in Anwesenheit von Katalysatoren der Gruppe Ib, IIb und VIII des Periodensystems der Elemente.

Aus der US-A-2 856 413 ist die Spaltung von Glycerincarbonat zu Glycidol ist an Phosphaten, Pyrophosphaten, Chloriden, Bromiden, Acetaten, Bicarbonaten und Carbonaten von Alkali- und Erdalkalimetallen bekannt.

Der vorliegenden Erfindung lag daher die Aufgabe zugrunde, ein Verfahren zu finden das ausgehend von Glycerin die Herstellung von Glycerincarbonat ermöglicht sowie deren Umsetzung zum Glycidol.

Demgemäß wurde ein neues und verbessertes Verfahren zur Herstellung von Glycerincarbonat gefunden, welches dadurch gekennzeichnet ist, daß man Glycerin mit Kohlenmonoxid und Sauerstoff in Gegenwart von Katalysatoren der I., II. und VIII. Nebengruppe des Periodensystems der Elemente bei Temperaturen von 0 bis 150°C umsetzt sowie deren Umsetzung zum Glycidol durch Reaktion bei Temperaturen von 80 bis 300°C und Drücken von 0,001 bis 5 bar an Alkali- und/oder Erdalkalimetallsalzen.

Das erfindungsgemäße Verfahren kann wie folgt durchgeführt werden:

Die Reaktion kann gestartet werden, indem man ein Kohlenmonoxid/Sauerstoff-Gasgemisch in die Reaktionsmischung einleitet. Die Zusammensetzung des Gasgemisches kann in weiten Grenzen beliebig gewählt werden. Statt Sauerstoff kann beispielsweise auch Luft verwendet werden oder das Gasgemisch gegebenenfalls mit einem Inertgas wie Stickstoff, Argon oder Kohlendioxid verdünnt werden. Im allgemeinen wird ein $CO/O_2$-Molverhältnis von 100 : 1 bis 0,5 : 1, bevorzugt 50 : 1 bis 1 : 1, besonders bevorzugt 20 : 1 bis 1,5 : 1 verwendet.

Die Reaktionsgase - Kohlenmonoxid und Sauerstoff - werden in die im Reaktor befindliche Flüssigkeit eingeleitet. Zur besseren Durchmischung und Dispergierung der Reaktionsgase im Reaktor kann bevorzugt zusätzlich mechanisch gerührt werden. Als Reaktortyp eignen sich Rührkessel bzw. Rührautoklaven oder Blasenreaktoren die zweckmäßigerweise beheizt werden können und gewünschtenfalls zusätzlich mit einer Rührvorrichtung versehen sein kann.

Die Umsetzung kann zweckmäßigerweise unter erhöhtem Druck, im allgemeinen bei einem Druck von 1 bis 100 bar, bevorzugt 2 bis 10 bar, besonders bevorzugt 2,5 bis 5 bar, gegebenenfalls in Anwesenheit eines inerten Lösungsmittels durchgeführt werden. Die Reaktionstemperatur beträgt im allgemeinen 0 bis 180°C, bevorzugt 80 bis 150°C, besonders bevorzugt 100 bis 140°C.

Die Umsetzung kann kontinuierlich oder diskontinuierlich durchgeführt werden. Bei der bevorzugten kontinuierlichen Betriebsweise werden die Reaktionsgase in der Regel im Überschuß verwendet und damit das nicht umgesetzte Gas im Kreis gefahren.

Als Katalysatoren eignen sich solche der Katalysatoren der I., II. und VIII. Nebengruppe des Periodensystems der Elemente wie Salze der Elemente Kupfer, Silber, Gold, Zink, Cadmium, Quecksilber, Eisen, Cobalt, Nickel, Ruthenium, Rhodium, Palladium, Osmium, Iridium und/oder Platin, bevorzugt Salze der Elemente Kupfer, Silber, Quecksilber, Eisen, Cobalt und/oder Nickel, besonders bevorzugt Salze der Elemente Kupfer und/oder Quecksilber.

Als Salze eignen sich Sulfate, Chloride, Bromide und Trifluoroacetate, bevorzugt Chloride, Bromide und Trifluoroacetate. Als Kupferkatalysatoren finden im erfindungsgemäßen Verfahren im allgemeinen einfache Kupfersalze wie Kupferhalogenide, Kupfer(I)sulfat, Kupfer(alkoxy)halogenide und Kupfer(I)-trifluoroacetat, bevorzugt Kupfer(I)halogenide wie Kupfer(I)chlorid und Kupfer(I)bromid sowie Kupfer(I)sulfat und Kupfer(I)-trifluoroacetat.

Als inerte Lösungsmittel eignen sich tert.-Amine wie $C_3$- bis $C_{20}$-tert.-Amine, beispielsweise Trimethylamin, Pyridin, Chinolin, $\alpha$-, $\beta$- und $\gamma$-Picolin, bevorzugt Pyridin und $\gamma$-Picolin, Nitrile wie Acetonitril, Propionitril und Benzonitril, Nitroverbindungen wie Nitrobenzol, bevorzugt Benzonitril oder Ester wie $C_2$- bis $C_{20}$-Carbonsäurealkylester, beispielsweise Essigsäuremethylester, Essigsäureethylester, Benzoesäureethylester, bevorzugt Benzoesäureethylester und Lactonen wie Butyrolacton, Harnstoffe wie Tetramethylharnstoff und Carbonate wie Propylencarbonat.

Die Umsetzung des Glycerincarbonates zum Glycidol kann bei Temperaturen von 100 bis 300°C, bevorzugt 125 bis 275°C, besonders bevorzugt 210 bis 250°C und Drücken von 0,001 bis 5 bar, bevorzugt 0,005 bis 2 bar, besonders bevorzugt 0,01 bis 1 bar an Alkali- und/oder Erdalkalimetallsalzen beispielsweise in einer Destillationsapparatur durchgeführt werden.

Als Alkali- und Erdalkalimetallsalze eignen sich Halogenide wie Fluoride, Chloride, Bromide und Iodide, Phosphate, Monohydrogenphosphate, Dihydrogenphosphate, Pyrophosphate, Sulfate, Borate, Acetate, Car-

EP 0 582 201 A2

bonate und Bicarbonate, bevorzugt Fluoride, Iodide, und Sulfate, besonders bevorzugt Fluoride, Sulfate und Borate. Im einzelnen seien genannt:

Lithiumfluorid, Natriumfluorid, Kaliumfluorid, Rubidiumfluorid, Cäsiumfluorid, Beryliumfluorid, Magnesiumfluorid, Calciumfluorid, Strontiumfluorid, Bariumfluorid, Lithiumchlorid, Natriumchlorid, Kaliumchlorid, Rubidiumchlorid, Cäsiumchlorid, Beryliumchlorid, Magnesiumchlorid, Calciumchlorid, Strontiumchlorid, Bariumchlorid, Lithiumbromid, Natriumbromid, Kaliumbromid, Rubidiumbromid, Cäsiumbromid, Beryliumbromid, Magnesiumbromid, Calciumbromid, Strontiumbromid, Bariumbromid, Lithiumiodid, Natriumiodid, Kaliumiodid, Rubidiumiodid, Cäsiumiodid, Beryliumiodid, Magnesiumiodid, Calciumiodid, Strontiumiodid, Bariumiodid, Lithiumsulfat, Natriumsulfat, Kaliumsulfat, Rubidiumsulfat, Cäsiumsulfat, Beryliumsulfat, Magnesiumsulfat, Calciumsulfat, Strontiumsulfat, Bariumsulfat, Lithiumborat, Natriumborat, Kaliumborat, Rubidiumborat, Cäsiumborat, Beryliumborat, Magnesiumborat, Calciumborat, Strontiumborat, Bariumborat, Lithiumacetat, Natriumacetat, Kaliumacetat, Rubidiumacetat, Cäsiumacetat, Beryliumacetat, Magnesiumacetat, Calciumacetat, Strontiumacetat, Bariumacetat, Lithiumcarbonat, Natriumcarbonat, Kaliumcarbonat, Rubidiumcarbonat, Cäsiumcarbonat, Beryliumcarbonat, Magnesiumcarbonat, Calciumcarbonat, Strontiumcarbonat, Bariumcarbonat, Lithiumphosphat, Natriumphosphat, Kaliumphosphat, Rubidiumphosphat, Cäsiumphosphat, Beryliumphosphat, Magnesiumphosphat, Calciumphosphat, Strontiumphosphat und Bariumphosphat, bevorzugt Lithiumfluorid, Natriumfluorid, Kaliumfluorid, Rubidiumfluorid, Cäsiumfluorid, Beryliumfluorid, Magnesiumfluorid, Calciumfluorid, Strontiumfluorid, Bariumfluorid, Lithiumiodid, Natriumiodid, Kaliumiodid, Rubidiumiodid, Cäsiumiodid, Beryliumiodid, Magnesiumiodid, Calciumiodid, Strontiumiodid, Bariumiodid, Lithiumsulfat, Natriumsulfat, Kaliumsulfat, Rubidiumsulfat, Cäsiumsulfat, Berylliumsulfat, Magnesiumsulfat, Calciumsulfat, Strontiumsulfat, Bariumsulfat, besonders bevorzugt Lithiumfluorid, Natriumfluorid, Kaliumfluorid, Rubidiumfluorid, Cäsiumfluorid, Magnesiumfluorid, Calciumfluorid, Strontiumfluorid und Bariumfluorid, Lithiumsulfat, Natriumsulfat, Kaliumsulfat, Rubidiumsulfat, Cäsiumsulfat, Magnesiumsulfat, Calciumsulfat, Strontiumsulfat und Bariumsulfat.

Das Glycerincarbonat sowie das Glycidol eignen als Zwischenprodukte für die Herstellung von Polymeren, z.B. Epoxiharze aus Glycidylestern von Polycarbonsäuren (Helv. Chim. Acta, 60, 1845 (1977)) oder Glycidylmethacrylat als Polymervernetzer (Helv. Chim. Acta, 60, 1845 (1977))

Beispiele

Beispiel 1

Oxidativen Carbonylierung von Glycerin

138 g (1,5 mol) Glycerin und 14,9 g Kupfer(I)chlorid wurden in einem 250 ml Glasrührautoklav vorgelegt und auf 110 °C erhitzt. Nachdem die Reaktionstemperatur erreicht war, wurde bei 6 bar Gesamtdruck einen Gemisch aus 80 % CO und 20 % $O_2$ durchgeleitet (100 ml/min bei Normaldruck). Nach 63 hr wurde die Reaktion unterbrochen und der Austrag mittels GC analysiert. Der Umsatz lag bei 47 % und Glycerincarbonat war das einzige erkennbare Produkt.

Beispiel 2

Oxidative Carbonylierung von Glycerin in inerten Lösungsmittel

230 g (0,25 mol) Glycerin, 150 g Nitrobenzol und 2,5 g (0,025 mol) Kupfer(I)chlorid wurden in einem 250 ml Glasrührautoklav vorgelegt und auf 130 °C erhitzt. Nachdem die Reaktionstemperatur erreicht war, wurde bei 8 bar Gesamtdruck einen Gemisch aus 95 % CO und 5 % $O_2$ durchgeleitet (100 ml/min bei Normaldruck). Nach 20 hr wurde die Reaktion unterbrochen und der Austrag mittels GC analysiert. Der Umsatz lag bei 96 % und Glycerincarbonat war das einzige erkennbare Produkt. Vergleichbaren Ergebnissen könnten auch mit Butyrolacton, Tetramethylharnstoff, N,N-Dimethylimidazol-2-onund Propylencarbonat als Lösungsmittel erzielt werden.

Beispiel 3

Herstellung von Glycidol

15 g Glycerincarbonat und 1,5 g Katalysator wurden in einer kleinen Destillationsapparatur vorgelegt und auf 10 bis 40 mbar evakuiert. Das Gemisch wurde in ein 200 °C heißes Ölbad eingetaucht. Die flüchtigen Reaktionsprodukte wurden mittels GC untersucht. Die erzielten Selektivitäten für Glycidol sind in Tabelle 1 zusammengefaßt. Als Nebenprodukt findet man Hydroxyaceton.

Tabelle 1

Selektivitäten in %

|  | F | Cl | Br | J | $H_2PO_4$ | $HPO_4$ | $PO_4$ | $SO_4$ | $B_4O_7$ |
|---|---|---|---|---|---|---|---|---|---|
| Li | 93 | 54 | 48 | 30 |  |  |  |  |  |
| Na | 99 | 89 | 90 | 52 | 99 | 98 | 95 | 99 | 70 |
| K | 87 | 87 | 90 | 64 | 91 |  |  |  |  |
| Rb | 81 | 86 | 83 | 73 |  |  |  |  |  |
| Cs | 82 | 82 | 84 | 84 |  |  |  |  |  |
| Mg |  | 16 |  |  |  |  |  |  |  |
| Ca | 98 | 30 |  |  |  |  |  |  |  |
| Ba | 50 |  |  |  |  |  |  |  |  |

Beispiel 4

Kontinuierliche Herstellung von Glycidol

In einen senkrechten, beheizten Glasrohr (∅ = 2 cm, H = 20 cm) wurden 2 g Natriumsulfat vorgelegt und die Apparatur auf 230°C beheizt und auf ca. 100 mbar evakuiert. Glycerincarbonat wurde dann von unten mittels eine HPLC-Pumpe zudosiert (0,5 mm/min). Die gasförmigen Produkte die oben aus dem Reaktor entströmten wurden auskondensiert und mittels GC analysiert. Der Glycerincarbonatumsatz lag bei 65 %, während die Selektivität zur Glycidol ca. 92 % war. Die Nebenprodukte waren fast ausschließlich nichtflüchtige Polymere, die im Reaktor zurückblieben.

**Patentansprüche**

1. Verfahren zur Herstellung von Glycerincarbonat, dadurch gekennzeichnet, daß man Glycerin mit Kohlenmonoxid und Sauerstoff in Gegenwart von Katalysatoren der I., II. und VIII. Nebengruppe des Periodensystems der Elemente bei Temperaturen von 0 bis 180°C umsetzt.

2. Verfahren zur Herstellung von Glycerincarbonat nach Anspruch 1, dadurch gekennzeichnet, daß man als Katalysatoren Salze der Elemente Kupfer, Silber, Gold, Zink, Cadmium, Quecksilber, Eisen, Cobalt, Nickel, Ruthenium, Rhodium, Palladium, Osmium, Iridium und/oder Platin einsetzt.

3. Verfahren zur Herstellung von Glycerincarbonat nach Anspruch 1, dadurch gekennzeichnet, daß man als Katalysatoren Chloride, Bromide oder Trifluoroacetate der Elemente Kupfer, Silber, Gold, Zink, Cadmium, Quecksilber, Eisen, Cobalt, Nickel, Ruthenium, Rhodium, Palladium, Osmium, Iridium und/oder Platin einsetzt.

4. Verfahren zur Herstellung von Glycerincarbonat nach Anspruch 1, dadurch gekennzeichnet, daß man als Katalysatoren Kupferchloride einsetzt.

5. Verfahren zur Herstellung von Glycerincarbonat nach Anspruch 1, dadurch gekennzeichnet, daß man die Umsetzung bei Temperaturen von 80 bis 150°C durchführt.

6. Verfahren zur Umsetzung von Glycerincarbonat, hergestellt nach Anspruch 1 zum Glycidol, dadurch gekennzeichnet, daß man die Reaktion bei Temperaturen von 80 bis 300°C und Drücken von 0,001 bis 5 bar an Alkali- und/oder Erdalkalimetallsalzen durchführt.